# EUROPÄISCHE PATENTSCHRIFT

(11) **EP 0 429 611 B1**
(45) Veröffentlichungstag und Bekanntmachung des Hinweises auf die Patenterteilung: **28.02.2001**
(21) Anmeldenummer: 90909048.2
(22) Anmeldetag: 16.06.1990
(51) Int. Cl.: C07D 207/452, G01N 33/535, G01N 33/547

(54) **AMINOALKYLMALEIMIDE UND DAVON ABGELEITETE HAPTEN- UND ANTIGENDERIVATE SOWIE KONJUGATE MIT PEPTIDEN ODER PROTEINEN**
AMINOALKYL MALEIMIDES AND HAPTENE AND ANTIGEN DERIVATIVES THEREOF AND CONJUGATES WITH PEPTIDES OR PROTEINS
AMINOALKYLMALEIMIDES ET DERIVES HAPTENES ET ANTIGENES ISSUS DE CEUX-CI ET PRODUITS CONJUGUES AVEC DES PEPTIDES OU DES PROTEINES

(30) Priorität: 19.06.1989 DE 3919915
(43) Veröffentlichungstag der Anmeldung: 05.06.1991
(73) Patentinhaber: Roche Diagnostics GmbH, 68298 Mannheim (DE)
(72) Erfinder: HUBER, Erasmus, D-8911 Unterfinning (DE); KLEIN, Christian, D-8120 Weilheim (DE); BATZ, Hans-Georg, D-8132 Tutzing (DE); ZINK, Bruno, D-8114 Uffing (DE)
(86) Internationale Anmeldenummer: EP9000957
(87) Internationale Veröffentlichungsnummer: WO9015798

(56) Entgegenhaltungen:
- EP-A- 0 142 193
- EP-A- 0 158 291
- EP-A- 0 178 125
- DE-A- 2 310 118
- CHEMICAL ABSTRACTS, Band 93, Nr. 19, 10. November 1980, (Columbus, Ohio, US), P. SINGH et al.: "A Solid Support for Affinity Chromatography that Covalently Binds Thiol Groups via a Cleavable Connector Arm", siehe seiten 294* Zusammenfassung 182140w, & Arch. Biochem. Biophys. 1980, 203(2), 774-9*

## Beschreibung

Die Erfindung betrifft neue Aminoalkylmaleimide der allgemeinen Formel I 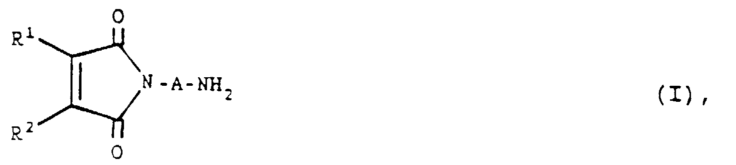 in der R₁ und R₂ gleich oder verschieden sind und Wasserstoff oder eine C₁-C₄-Alkylqruppe darstellen, und A eine geradkettige oder verzweigte, gesättigte oder ungesättigte, gegebenenfalls durch ein Sauerstoff- oder Schwefelatom oder eine Carbonylgruppe unterbrochene Alkylenkette mit 2 bis 6 Kohlenstoffatomen darstellt, sowie deren entsprechenden Säureadditionssalze, mit Ausnahme der Verbindung N-6-Aminohexylmaleimid.

Ferner bezieht sich die vorliegende Erfindung auf die aus Verbindungen der allgemeinen Formel I und immunologischen Bindungspartner gebildeten Amidoalkylmaleimid-Derivate der allgemeinen Formel II 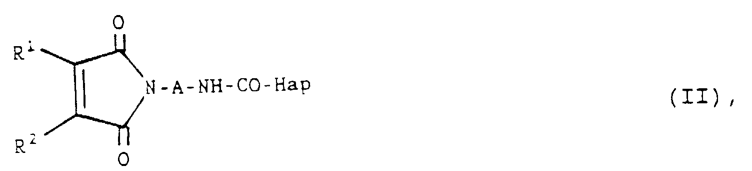 in der R₁, R₂ und A die oben angegebenen Bedeutungen haben und Hap, der aus einem eine oder mehrere Carboxylgruppen bzw. aktivierte Carboxylgruppen tragenden immunologischen Bindungspartner durch Amidierung gebildete Rest ist. Als immunologische Bindungspartner kommen beispielsweise Haptene in Frage.

Außerdem betrifft die Erfindung immunologische Konjugate, die durch Umsetzung von Verbindungen der allgemeinen Formel II mit Peptiden oder Proteinen hergestellt werden können. Diese Konjugate werden im folgenden durch den Begriff Hapten-Peptid- oder Hapten-Protein-Konjugat umschrieben.

Gegenstand der vorliegenden Erfindung sind auch Verfahren zur Herstellung der Verbindungen der allgemeinen Formeln I und II sowie der Peptid- und Protein-Konjugate, und die Verwendung dieser Konjugate in diagnostischen Bestimmungsmethoden, insbesondere in Immunoassays.

In vielen biochemischen Techniken ist die Verknüpfung von zwei sich funktional unterscheidenden Komponenten bzw. Verbindungen von grundlegender Bedeutung. So werden beispielsweise auf dem Gebiet der Affinitätschromatographie an eine feste unlösliche Trägermatrix verschiedenartige Liganden gebunden. Diese Liganden sind in der Lage, die in einer Probe enthaltenen und für diesen betreffenden Liganden spezifischen Substanzen zu erkennen und zu binden. Dadurch ist eine gezielte Abtrennung der in der Probe enthaltenen Verbindungen möglich. Als Liganden kommen beispielsweise Antikörper oder Enzyme in Frage, die ein Antigen oder ein Substrat spezifisch binden.

Zur Verknüpfung von Komponenten im obigen Sinne werden häufig bifunktionelle Reagenzien, die auch als Linker bezeichnet werden, eingesetzt. Diese enthalten in der Regel zwei chemisch reaktive Gruppen, die mit bestimmten Resten der miteinander zu verknüpfenden Komponenten möglichst spezifisch reagieren. Generell unterscheidet man hierbei zwischen hetero-bifunktionellen Reagenzien einerseits, wie z.B. gamma-Maleimido-buttersäure-N-hydroxy-succinimid oder m-Maleimido-benzoyl-N-hydroxysuccinimidester (MBS), und homo-bifunktionellen Reagenzien andererseits, wie z.B. N,N'-Bis-(3-maleimidopropionyl)-2-hydroxy-1,3-propandiamin. Die heterobifunktionellen Reagenzien besitzen zwei chemisch unterschiedliche reaktive Gruppen, wie beispielsweise im obigen Fall die Maleimido- und die N-Hydroxy-succinimido-Gruppe. Während die Maleimidogruppe sehr spezifisch mit sulfhydrylhaltigen Verbindungen reagiert, eignet sich die Hydroxysuccinimidogruppe bevorzugt zur Reaktion mit Verbindungen, die eine Aminogruppe enthalten. Auf diese Weise lassen sich auf elegante Art und Weise zwei Komponenten bzw. Verbindungen miteinander verknüpfen, wobei die eine beispielsweise eine Sulfhydrylgruppe enthält und die andere eine Aminogruppe. In analoger Weise dienen die homobifunktionellen Reagenzien, die zwei chemisch gleichartige reaktive Gruppen enthalten, zur Verknüpfung von Komponenten mit identischen funktionellen Gruppen, wie beispielsweise im obigen Fall von Sulfhydrylgruppen.

Je nach Eigenschaft der miteinander zu verknüpfenden Komponenten kommen demnach eine Reihe von solchen bifunktionellen Reagenzien zum Einsatz (Kia-ki Han et al., Int. J. Biochem. 16 (2), 129 - 145 (1984) und R.E. Feeney, Int. J. Peptide Protein Res. 29, 1987, 145 - 161). So ist beispielsweise in Arch. Biochem. Biophys. 203, 774 (1980) die Verwendung von N-6-Aminohexylmaleimid zur Herstellung einer modifizierten Agarose-Festphase für die Affinitätschromatographie beschrieben.

Aus dem Stand der Technik, wie z.B. der europäischen Patentanmeldung EP-A-0,142,193, ist ferner die Herstellung von Immunogenen bekannt, wobei geeignete Antigene, wie Polypeptide, Oligosaccharide oder Oligonukleodtide, an reaktive Gruppen von hydophoben Verbindungen, wie z.B. Phospholipide, gebunden werden, die durch mindestens eine Gruppe von Glykosiden, vorzugsweise Saponine, mit einem hydrophilen und einem hydrophoben Teil komplexiert werden. Im speziellen ist in Beispiel 4 dieser europäischen Patentanmeldung ein β-Endorphin-Derivat beschrieben, in dem eine Aminosäure dieses Dodecapeptids durch eine Maleimido-butylamin-gruppe modifiziert ist. Die Anmeldung enthält jedoch keinerlei Angaben, wie ein derartiges Derivat hergestellt werden kann.

Ein weiteres Anwendungsgebiet bifunktioneller Reagenzien ist die Herstellung von Reagenzien für die klinische Diagnostik. Hierbei werden in zunehmendem Maße Bestimmungsverfahren nach dem Prinzip der Immunoassays durchgeführt, die sich durch besonders hohe Empfindlichkeit auszeichnen. Dabei werden oft Konjugate z.B. aus einem Markierungsenzym und einer zu bestimmenden Substanz, dem sogenannten Analyten, bzw. einer einen Antikörper bindefähigen Substanz, dem sogenannten Antigen, eingesetzt. Diese Konjugate lassen sich vorzugsweise durch den Einsatz von bifunktionellen Reagenzien herstellen.

Ein weiteres Beispiel aus jüngster Zeit ist die auf dem Gebiet der homogenen Immunoassays immer mehr an Bedeutung gewinnende sogenannte CEDIA-Technik (Clinical Chemistry 32/9, 1637 - 1641 (1986); US-4,708,929). Bei dieser diagnostischen Bestimmungsmethode wird ebenfalls von dem Prinzip der Verknüpfung von zwei Verbindungen Gebrauch gemacht. Dabei wird ein Analyt, der im allgemeinen ein Hapten oder ein Antigen sein kann, an eine enzymatisch inaktive Vorläuferstufe des Enzyms β-Galactosidase angeknüpft. Diese inaktive Vorläuferstufe ist ein Peptid und wird in der oben zitierten Literatur auch als Enzymdonor bezeichnet. Mittlerweile sind eine Reihe von verschiedenen Peptiden als Enzymdonoren bekannt, wie z.B. ED4.

Die Durchführung dieses Tests beruht auf folgendem Prinzip: Das Enzym β-Galactosidase besteht aus vier Untereinheiten, die wiederum aus einer größeren Polypeptidkette, die auch als Enzymakzeptor EA bezeichnet wird, und einer kleineren Peptidkette, Enzymdonor ED genannt, aufgebaut ist. Enzymakzeptor und Enzymdonor sind beide enzymatisch inaktiv, können sich aber spontan zu einem aktiven Tetramer zusammenlagern. Aufgrund der außerordentlich geringen Dissoziationskonstanten des aus Enzymdonor und Enzymakzeptor gebildeten aktiven Enzyms ist die Menge des gebildeten Enzyms direkt proportional zur Menge des vorhandenen Enzymdonors bzw. -akzeptors. Bei der Durchführung von Immunoassays macht man sich die Eigenschaft nun dadurch zu Nutzen, in dem man anstelle des natürlichen Enzymdonors einen chemisch modifizierten Enzymdonor einsetzt. Dieser zeichnet sich dadurch aus, daß ein Analyt kovalent an funktionelle Gruppen des Enzymdonors geknüpft ist. Als Analyte kommen beispielsweise Haptene oder Antigene in Frage.

Das bei der immunologischen Bestimmungsmethode verwendete Testsystem enthält außerdem einen den jeweiligen Analyten spezifisch Immunogen erkennenden Antikörper. Hierbei differenziert der Antikörper in der Regel nicht zwischen freien und kovalent an den Enzymdonor gebundenen Analyten. Der Antikörper ist in einer Menge vorhanden, die ausreicht, um die im Testsystem vorliegenden mit dem Analyten modifizierten Enzymdonor-Konjugate vollständig zu binden. Dadurch wird die spontane Assoziation des Enzymdonors und des Enzymakzeptors zum aktiven Enzym verhindert.

Die Reaktion zur Bestimmung der Konzentration eines Analyten wird nun derart durchgeführt, daß eine definierte Menge der zu bestimmenden Probe zu dem oben beschriebenen Testsystem zugefügt wird. Da es sich bei diesem Reaktionstyp um einen kompetitiven Test handelt, binden die vorhandenen Antikörper, die gegen den Analyten gerichtet sind, sowohl die aus der Probe kommenden freien Analyten als auch einen Teil der mit dem Analyten markierten Enzymdonoren. Der durch die Verdrängungsreaktion durch den freien Analyten ausgelöste freie Anteil des markierten Enzymdonors assoziiert dann spontan mit dem Enzymakzeptor zum enzymatisch aktiven β-Galaktosidase-Tetramer, dessen Konzentration direkt proportional zu der Konzentration des aus der Probe stammenden Analyten ist. Die Menge bzw. die volumenspezifische Aktivität des durch diese spontane Assoziation gebildeten Enzyms β-Galactosidase wird durch Hydrolyse eines geeigneten Enzymsubstrates, beispielsweise von o-Nitrophenyl-β-D-galactopyranosid oder Chlorphenolrot-β-D-galactopyranosid und Freisetzung des entsprechenden Chromophors gemessen.

Bei der oben dargestellten erforderlichen Verknüpfung zwischen Immunogen (Analyt) und Enzymdonor besteht das Problem, daß die Modifizierung des Enzymdonors nur an solchen Stellen im Molekül erfolgen darf, die für die Erkennung zwischen Enzymdonor und Enzymakzeptor nicht von Bedeutung sind. Ansonsten wäre die Assoziation zum enzymatisch aktiven Enzymkomplex gestört und der gemessene Wert für das zu bestimmende Hapten würde ein falsches Ergebnis vortäuschen.

Es ist literaturbekannt, daß als Hapten-Enzymdonor-Konjugatbeispielsweise Maleimidyl-benzcarbamyl-digoxigenin eingesetzt werden kann (Clin.Chem. 32, 1637 (1986)). Die Bindung des Haptens Digoxigenin an den Enzymdonor erfolgt durch Umsetzung mit 3-Maleimidobenzoesäure-isocyanat. Die Verknüpfung seitens des Enzymdonors mit dem oben gebildeten Digoxigenin-Derivat erfolgt durch Reaktion einer Sulfhydrylgruppe der Aminosäure Cystein am Enzymdonor mit der Maleimidogruppe.

Das in dem oben genannten Fall eingesetzte bifunktionelle Reagens hat jedoch den Nachteil, daß einerseits die vorhandene Urethan-Bindung hydrolyseempfindlich ist und das Reagenz somit nicht hinreichend stabil genug ist, und daß ferner durch den vorhandenen Benzcarbamylrest ein Teil der Bindungsaktivität der Antikörper gegen den Linker selbst, d. h. gegen das Brückenmolekül zwischen Hapten und Enzymdonor im Immunogen gerichtet ist. Dies bedeutet, daß bei der Antigen-Antikörper-Reaktion häufig mit Kreuzreaktivitäten zu rechnen ist, die im immunologischen Test zu Ungenauigkeiten bei der Bestimmung führen.

Außerdem besteht das Problem, daß die verwendeten bifunktionellen Reagenzien möglichst stabil und einfach herstellbar sein sollten. Ferner ist es wünschenswert, daß sie mit Haptenen oder Antigenen unter möglichst milden Bedingungen reagieren, und somit die zur Immunisierung und Gewinnung von Antikörpern als Immunogene verwendbaren Hapten-Peptid-Derivate auf schonende und einfache Weise hergestellt werden können.

Heterobifunktionelle Reagenzien mit einer Maleimidogruppe haben jedoch häufig den Nachteil, daß sie sehr reaktiv sind und leicht zu Maleinsäure-derivaten hydrolysieren. Aufgrund ihrer hohen Reaktivität sind sie oft wenig spezifisch für Sulfhydrylgruppen, insbesondere wenn sie in stöchiometrischen Mengen eingesetzt werden. Diese geringe Spezifität kann zu komplexen Reaktionsmischungen führen, die entstehen, wenn die Maleimidogruppe mit anderen reaktiven Gruppen der zu verknüpfenden Peptide oder Proteine, wie z.B. mit Aminen, reagieren. Derartige Nebenreaktionen führen zu unerwünschten Reaktionsmischungen, die häufig schwierig zu trennen sind. Höchste Reinheit der hergestellten Konjugate ist jedoch erforderlich, um durch Immunisierung Antikörper mit möglichst hoher Spezifität und geringer Kreuzreaktivität zu erhalten.

Es bestand daher die Aufgabe, neue bifunktionelle Reagenzien zur Verfügung zu stellen, die die oben genannten Nachteile nicht aufweisen.

Es wurde nun überraschenderweise gefunden, daß die erfindungsgemäßen Verbindungen der Formel I als heterobifunktionelle Reagenzien vorteilhaft eingesetzt werden können. Insbesondere hat sich gezeigt, daß die zwischen der Maleimido- und der Aminogruppe befindliche Gruppe A für eine geringe Kreuzreaktivität der mit Hilfe der Hapten-Peptid-Konjugate hergestellten Antikörper verantwortlich ist. Dies trifft insbesondere zu für die kurzkettigen Derivate mit einer Kettenlänge in A von 2-5, insbesondere 2, 3 oder 4 Atomen.

R¹ und R² in der Formel I können gleich oder verschieden sein und jeweils ein Wasserstoffatom oder eine C₁-C₄-Alkylgruppe, wie z.B. die Methyl-, Ethyl- oder Isopropylgruppe bedeuten. Bevorzugt stellen R¹ und R² jedoch ein Wasserstoffatom dar.

Die Gruppe A bedeutet eine geradkettige oder verzweigte, gesättigte oder ungesättigte Alkylengruppe mit 2-6, vorzugsweise 2-4 C-Atomen, die gegebenenfalls durch ein Sauerstoff- oder Schwefelatom oder durch eine Carbonylgruppe unterbrochen sein kann. In diesem Sinne kommen beispielsweise die folgenden Bedeutungen in Frage: A=-(CH₂)ₙ-; -(CH₂)ₘ-CH(CH₃)-(CH₂)ₒ-; -(CH₂)ₘ-C(CH₃)₂-(CH₂)o-; -(CH₂)ₘ-X-(CH₂)ₒ- mit X=O,S,CO; -(CH₂)ₘ-CH=CH- mit n=2-6 und m=o=l-4 und m+o=2-5. Bevorzugt bedeutet A die Gruppen -(CH₂)ₙ- mit n=2,3,4 oder 5 und -CH₂-O-CH₂-, -CH₂-S-CH₂-, -CH₂-CO-CH₂-.

Säureadditionssalze der Amine sind Salze mit organischen oder anorganischen Säuren, wie z.B. Essigsäure oder Salzsäure.

Verbindungen der Formel I können beispielsweise dadurch hergestellt werden, indem man ein Diamin der allgemeinen Formel III

H₂N-A-NH₂ (III),

in der A die oben angegebene Bedeutung hat, in eine Verbindung der allgemeinen Formel IV

H₂N-A-NH-Y (IV),

überführt, wobei Y eine für die Aminogruppe geeignete leicht abspaltbare Schutzgruppe, beispielsweise die tert.-Butyloxycarbonylgruppe darstellt, und anschließend die Verbindung der allgemeinen Formel IV mit einer Verbindung der allgemeinen Formel V 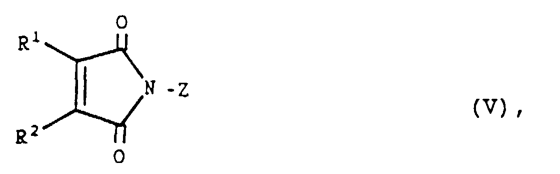 in der R₁ und R₂ die oben angegebenen Bedeutungen haben und Z eine reaktive Gruppe bedeutet, nach an sich bekannten Verfahren umsetzt, und anschließend die Schutzgruppe Y wieder abspaltet.

Die Umsetzung der Diamine der allgemeinen Formel III mit geeigneten Schutzgruppenreagenzien, wie z.B. Di-tert.-butyl-dicarbonat, tert.-Butoxycarbonylchlorid oder N-(tert.-Butoxy-carbonyloxy)phthalimid, erfolgt in Lösungsmittel, wie z. B. Dioxan, Tetrahydrofuran, Dimethylformamid oder Ethanol bei Temperaturen von - 20 °C bis + 100 °C, vorzugsweise bei 0 °C bis + 25 °C nach an sich bekannten Methoden.

Die Umsetzung der Verbindungen der Formel IV mit Verbindungen der Formel V werden vorzugsweise in basischen Lösungen bei Temperaturen von - 20 °C bis + 80 °C durchgeführt. Als reaktive Gruppe Z kommen solche in Frage, die sich durch Umsetzung mit Aminen leicht abspalten lassen, wie z.B. Alkoxycarbonylgruppen, beispielsweise die Ethoxycarbonylgruppe.

Die neuen Verbindungen der Formel I finden als heterobifunktionelle Reagenzien insbesondere zur Herstellung von Konjugaten Verwendung. Als Konjugate kommen beispielsweise Hapten-Peptid-, Hapten-Protein-, Antigen-Peptid-, Antigen-Protein- oder Antikörper-Effektor-Konjugate in Frage. Im Fall der Antikörper-Effektor-Konjugate wird beispielsweise ein Antikörper kovalent an ein Effektormolekül gebunden, wobei der Effektor ein Analyt, Wirkstoff, Toxin oder auch ein signalproduzierendes Enzym sein kann.

Ein weiterer Gegenstand der vorliegenden Erfindung sind Verbindungen der Formel II, in der Hap der aus einem eine oder mehrere Carboxylgruppen oder Carboxylderivate tragenden Hapten durch Amidierung gebildete Rest ist, wobei das Hapten Phenobarbital, Diphenylhydantoin, Carbamazepin, Valproeinsäure, Thyroxin (T4), Triiodthyronin (T3), Östron, Östradiol, Progesteron, Testosteron, Aldosteron, Folsäure, Methyltetrahydrofolsäure oder Cyancobalamin (Vitamin B12), ein Steroidhormon oder ein Xanthinderivat darstellt R¹ und R 2 gleich oder verschieden sind und eine C1-C4-Alkylgruppe oder ein Wasserstoffatom bedeuten, und A eine geradkettige oder verzweigte, gesättigte oder ungesättigte, gegebenenfalls durch ein Sauerstoff- oder Schwefelatom oder Carbonylgruppe unterbrochene Alkylengruppe mit 2-6 C-Atomen darstellt.

Zur Herstellung der Amidoalkylmaleimid-Derivate der allgemeinen Formel II kommen die oben unter Hap definierten Haptens in Frage. Für die Umsetzung der Maleimide der allgemeinen Formel I mit geeigneten Hap-Derivaten ist es zweckmäßig, die in Hap vorhandene Carboxylgruppe zunächst durch Überführung in ein reaktives Derivat zu aktivieren. Dies geschieht beispielsweise durch Umsetzung mit N-Hydroxysuccinimidester in Gegenwart eines Kondensationsmittels, wie z.B. N,N'-Dicyclohexylcarbodiimid. Der auf diese Weise hergestellte Hap-Carbonsäure-N-hydroxysuccinimidester wird dann direkt mit dem Maleimidderivat der allgemeinen Formel I in hoher Ausbeute zu den entsprechenden Maleimid-Hapten-Derivaten der allgemeinen Formel II umgesetzt. Es ist jedoch prinzipiell auch möglich, andere Aktivierungsgruppen der Carboxylgruppe zu verwenden, wie z.B. die Imidazolyl-, Hydroxybenzotriazolyl-, p-Nitrophenyl- oder Isobutoxycarbonlygruppen.

In bevorzugten Ausführungsformen der erfindungsgemäß Verbindungen der allgemeinen Formel II ist der immunologische Bindungspartner ein Hapten, wie z. B. ein Steroidhormon oder ein Xanthinderivat, beispielsweise Cortisol, Theophyllin oder Digoxigenin. Prinzipiell können jedoch alle diejenigen Haptene eingesetzt werden, die eine reaktive Carboxylgruppe tragen. Die Carboxylgruppe kann in freier Form vorliegen oder in Form von aktivierten Derivaten, wie beispielsweise Anhydriden, Estern oder Amiden. Es ist jedoch auch möglich, solche Haptene als Bindungspartner einzusetzen, die ursprünglich keine Carboxylgruppe enthielten, wenn durch chemische Modifizierung die entsprechende Carboxylgruppe nachträglich in das Hapten eingeführt wird. Entsprechende Modifizierungsreaktion sind aus dem einschlägigen Stand der Technik bekannt.

In einer weiteren Ausführungsform der Erfindung werden Verbindungen der allgemeinen Formel II zur Verfügung gestellt, in denen das Hapten ein Steroidhormon ist. Der Nachweis von Steroidhormonen, wie z.B. östrogen, Testosteron, Cortisonen und Herzglycosiden, denen alle das Steroidgrundgerüst gemeinsam ist, spielt in der Diagnostik eine bedeutende Rolle. Die Zurverfügungstellung von Antikörpern gegen Steroidhormone bzw. gegen die entsprechenden Hapten-Peptid-Konjugate für die Durchführung von Immunoassays ist daher wünschenswert.

Es hat sich erwiesen, daß durch die Derivatisierung der Haptene zu den Verbindungen der allgemeinen Formel II das Epitop des Haptens nicht beeinträchtigt wird, so daß ein gegen das jeweilige in der zu bestimmenden Probe vorhandene freie Hapten gerichteter Antikörper auch das entsprechend modifizierte Hapten-Peptid-Konjugat in gleichem Maße erkennt.

Ebenso hat sich gezeigt, daß die derivatisierten Hapten-Peptid-Konjugate, die durch Umsetzung von Verbindungen der Formel II mit sulfhydrylgruppenhaltigen Peptiden oder Proteinen erhalten werden, ohne weitere Beeinträchtigung der Bindungseigenschaften mit dem Enzymakzeptor EA zu den Tetramer-Komplexen der β-Galactosidase assoziieren. Diese wiederum zeigen durch die im Prinzip vorhandene Derivatisierung des Enzyms keine nachteilige Beeinflussung der enzymatischen Aktivität, und spalten die in Frage kommenden Substrate in gleicher Weise wie die unmodifizierte β-Galactosidase.

Bei der Verwendung für die Immunisierung werden die Hap-Derivate der allgemeinen Formel II eingesetzt. Das Haptenderivat wird in den zur Antikörperbildung geeigneten Organismus in Abständen mehrmals injiziert. Es kommt dabei zur Bildung von Antikörpern, die in sehr hohem Prozentsatz nun gegen das Hapten gerichtet sind, und keine Kreuzreaktivität mit den Verknüpfungsgruppierungen aufweisen. Die Antikörper werden dann in an sich bekannter Weise aus dem Organismus isoliert und gereinigt. Die erfindungsgemäßen Haptenderivate der allgemeinen Formel II eignen sich sowohl zur Immunisierung für die Herstellung polyklonaler Antikörper als auch zur Herstellung monoklonaler Antikörper.

Überraschenderweise gelingt es durch Verwendung der erfindungsgemäßen Haptenderivate der allgemeinen Formel II, Antikörper zu erhalten, die sehr wenig Kreuzreaktivität mit der Brückenverbindung aufweisen. Die erhaltenen Antikörper haben eine hohe Affinität zu dem Hapten. Bei der Verwendung der erfindungsgemäßen Haptenderivate zur Immunisierung werden hohe Antikörpertiter erhalten. Weiterhin sind die erfindungsgemäßen Hapten-Peptid-Konjugate besonders gut geeignet zum Einsatz in Immunoassays. Da die Antikörper zu dem Brückenmolekül keine oder eine nur sehr geringe Affinität aufweisen und daher das Hapten sehr spezifisch binden, erhält man mit diesen Konjugaten bei der Verwendung in Immunoassays sehr genaue Ergebnisse.

Die erfindungsgemäßen Haptenderivate der allgemeinen Formel II besitzen den Vorteil, daß sie einerseits die Struktur des freien Haptens weitgehend unverändert beinhalten, andererseits im Vergleich zu Derivaten, die z.B. aus dem Stand der Technik bekannt sind, im Brückenmolekül eine charakteristische Änderung aufweisen. Die Verwendung der erfindungsgemäßen Haptenderivate ermöglicht deshalb die Durchführung heterologer Linker-Techniken auf einfache und effektive Weise.

Die vorliegende Erfindung betrifft ferner Konjugate, die durch Umsetzung der Verbindungen der allgemeinen Formel II mit Peptiden und Proteinen hergestellt werden können. Als Peptide und Proteine kommen vorzugsweise solche in Frage, die eine Sulfhydylgruppe bzw. eine Cysteingruppe enthalten, die mit der Maleimidogruppe der Verbindungen der Formel II reagieren. Die Herstellung solcher Derivate erfolgt nach an sich bekannten Methoden der chemischen Modifizierung von Proteinen. Vorzugsweise werden jedoch Peptide, insbesondere die in der oben beschriebenen CEDIA-Technik verwendbaren Enzymdonatoren ED eingesetzt. Die Modifizierung von Peptiden oder Proteinen erfolgt nach an sich bekannten Verfahren in geeigneten Puffern, z.B. Phosphatpuffer, bei Temperaturen zwischen 0°c und 40°C, vorzugsweise bei 15-25°C, durch Inkubation mit Verbindungen der Formel II über einen Zeitraum von 1-24h. Die Herstellung verschiedener ED-Derivate ist aus US 4,708,929 bzw. Clin. Chem. 32 (9), 1986, 1637 - 1641 bekannt, bzw. sind bei Microgenics Corp., Concord, California (USA) erhältlich.

Gegenstand der Erfindung ist deshalb auch die Verwendung eines erfindungsgemäßen Hapten-Peptid-Konjugates bei der Durchführung von Immunoassays.

Die folgenden Beispiele erläutern die Erfindung anhand von konkreten Ausführungsbeispielen:

### Beispiel 1

a) N-(tert.-Butyloxycarbonyl)-ethan-1,2-diamin
   12 g (0.2 mol) Ethylendiamin werden in 200 ml Dioxan/Wasser (50/50 v/v) gelöst. Innerhalb von 1.5 h wird unter Rühren und Kühlung im Eisbad eine Lösung von 21.8 g (0.1 mol) Di-tert.-butyl-dicarbonat in 100 ml Dioxan zugetropft. Man läßt 1 h bei Raumtemperatur weiterrühren, dann wird mit 500 ml Wasser verdünnt und mit 1 l Essigester extrahiert. Die organische Lösung wäscht man dreimal mit je 200 ml Wasser und extrahiert mit zweimal je 300 ml 0.1 n HC1. Die vereinigten HCl-Phasen werden mit 2 n NaOH auf pH 10.0 gestellt und mit 600 ml Essigester extrahiert. Die organische Lösung wird nochmals mit 300 ml Wasser gewaschen, mit 20 g Na₂SO₄ getrocknet und im Wasserstrahlvakuum eingedampft.
   Ausbeute: 1.35 g zähes Öl (entspr. 8.4 % d.Th. bez.auf Di-tert.-butyl-dicarbonat).
   DC: Kieselgel, Methanol/Essigester (66/33 v/v), Besprühen mit Ninhydrin-Spray (Fa. Merck, Darmstadt); R_{f} = 0.14.
b) N-(tert.-Butyloxycarbonyl)pentan-1,5-diamin-Acetat
   20.4 g (0.2 mol) Pentan-1.5-diamin werden in 200 ml Dioxan/Wasser (50/50 v/v) gelöst. Innerhalb von 1.5 h wird unter Rühren und Kühlung im Eisbad eine Lösung von 21.8 g (0.1 mol) Di- tert.-butyl-dicarbonat in 100 ml Dioxan zugetropft. Man läßt 2 h bei 0°C und anschließend 18 h bei 20°C weiterrühren, dann wird mit 500 ml Wasser verdünnt und mit 1 l Essigester extrahiert. Die organische Lösung wäscht man zweimal mit je 200 ml Wasser, trocknet mit 50 g Na₂SO₄ und dampft am Rotationsverdampfer ein. Der halbfeste Rückstand wird mit 200 ml 10%iger Essigsäure digeriert und das ungelöste Bis-BOC-pentan-1,5-diamin abgesaugt. Man dampft das Filtrat ein und trocknet das verbleibende zähflüssige Produkt 4-6 h am Hochvakuum.
   Ausbeute: 11.6 g (entspr. 44% d. Th. bez. auf die Di-tert.-butyl-dicarbonat).
   DC: Kieselgel, Methanol/Chloroform/Ammniak 45/45/10 (v/v/v) Besprühen mit Ninhydrin-Spray (Fa. Merck, Darmstadt); R_{f}=0.65.

### Beispiel 2

a) N-(tert-Butyloxycarbonyl)-2-(N-maleinimido)ethylamin
   0.8 g (5 mmol) der nach Beispiel la hergestellten Verbindung löst man in 25 ml gesättigter Natriumbicarbonat-Lösung. Die Lösung wird über ein Falten-filter filtriert und auf 0°C gekühlt. Anschließend gibt man unter Rühren 0.84 g (5 mmol) N-(Ethoxycarbonyl)maleinimid (hergestellt nach der Methode von 0. Keller und J. Rudinger, Helv.Chim.Acta 58 (1975), 531-541) zu und läßt 15 min bei Raumtemperatur weiterrühren. Hierbei löst sich das N-(Ethoxycarbonyl)maleinimid nach kurzer Zeit vollständig auf, während die Titelverbindung im Verlauf der Umsetzung ausfällt. Es werden 40 ml THF zugegeben und 45 min bei Raumtemperatur weitergerührt. Danach stellt man mit 1 n HCl auf pH 6.0, extrahiert mit zweimal je 50 ml Essigester und trocknet den Extrakt mit 5 g Na₂SO₄. Nach Eindampfen im Wasserstrahlvakuum wird die Titelverbindung als farbloser, fester Rückstand erhalten.
   Ausbeute: 1.1 g (92 % d.Th.).
   DC: Kieselgel, Chloroform/Essigester (66/33 v/v), Besprühen mit 0.1 % KMnO₄-Lsg.; R_{f} = 0.50.
b) N-(tert.-Butyloxycarbonyl)-5-(N-maleinimido)pentylamin
   13.1 g (50 mmol) der nach Beispiel lb hergestellten Verbindung löst man in 250 ml gesättigter Natriumcarbonat-Lösung. Die Lösung wird über ein Faltenfilter filtiert und auf 0°C gekühlt. Nun gibt man unter Rühren 8.4 g (50 mmol) N-(Ethoxycarbonyl)maleinimid zu und läßt 15 min bei Raumtemperatur weiterrühren, wobei sich das N-(Ethoxycarbonyl)-maleinimid nach kurzer Zeit vollständig auflöst. Anschließend gibt 400 ml Tetrahydrofuran und zusätzlich 250 ml gesättigte Natriumcarbonat-Lösung zu und läßt 1 h weiterreagieren. Dann wird die Lösung mit 2 x 500 ml Essigester extrahiert, der Extrakt mit 500 ml Wasser gewaschen und mit 50 g Na₂So₄ getrocknet. Nach dem Eindampfen am Rotationsverdampfer erhält man das Produkt als zähes Öl das am Hochvakuum getrocknet wird.
   Ausbeute: 9.6 g (entspr. 68% d. Th.)
   DC: Kieselgel, n-Butanol/Eisessig/Wasser 40/10/50 (v/v/v), Besprühen mit 0.1% KMnO₄-Lsg.; R_{f}=0.85.

### Beispiel 3

a) 2-(N-Maleinimido)ethylamin-Hydrochlorid
0.96 g (4 mmol) der BOC-Aminoverbindung aus Beispiel 2a werden in 25 ml 2 m HCl in Dioxan gelöst und bei Raumtemperatur stehen gelassen. Innerhalb 30 - 60 min beginnt das Produkt 4 auszufallen. Man läßt 24 h bei Raumtemperatur stehen, dann saugt man das Kristallisat ab, wäscht mit ca. 10 ml Essigester nach und trocknet im Exsikkator über CaCl₂ und Paraffin.
Ausbeute: 0.58 g farbloses, feinkristallines Pulver (82 % d.Th.).
DC: Kieselgel, n-Butanol/Eisessig/Wasser (40/10/50 v/v/v), Besprühen mit Ninhydrin-Spray (Fa. Merck, Darmstadt) ; R_{f} = 0.22.

| | | | | | | | |
|---|---|---|---|---|---|---|---|
| C₆H₉ClN₂O₂ (176.60); | Ber. | C | 40.81 | H | 5.14 | N | 15.86 |
| | Gef. | C | 40.60 | H | 5.29 | N | 15.46 |

b) 5-(N-Maleinimido)pentylamin-Hydrochlorid
8.46 g (30 mmol) der nach Beispiel 2b hergestellten BOC-Aminoverbindung werden in 100 ml 2 m HCL in Dioxan gelöst und bei Raumtemperatur stehen gelassen. Innerhalb 30-60 min beginnt das Produkt auszufallen. Man läßt 24 h bei Raumtemperatur stehen, dann saugt man das Kristallisat ab, wäscht mit ca 50 ml Essigester nach und trocknet im Exsikkator über CaCl₂ und Paraffin.
Ausbeute: 4.7 g farbloses, feinkristallines Pulver (entspr. 72% d.Th.)
DC: Kieselgel, n-Butanol/Eisessig/Wasser (40/10/50 (v/v/v), Besprühen mit Ninhydrin-Spray (Fa. Merck, Darmstadt); R_{f}0.22

| | | | | | | | |
|---|---|---|---|---|---|---|---|
| C₉H₁₅ClN₂O₂(218.69); | Ber. | C | 49.43 | H | 9.91 | N | 12.81 |
| | Gef. | C | 49.19 | H | 9.99 | N | 12.59 |

### Beispiel 4 Herstellung von Hapten-Carbonsäure-Derivaten

Die folgenden Haptencarbonsäuren wurden nach Literaturvorschriften hergestellt:
a) Cortisol-3-(O-carboxymethyl)-oxim
   aus Cortisol und Carboxymethylhydroxylamin-hemihydrochlorid in Ethanol
   Lit.: A. Tsuji et al., Steroids 24 (1974), 739-51 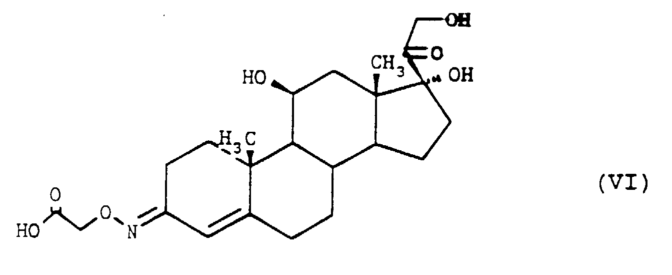
b) 3-O-Succinyl-Digoxigenin
   aus Digoxigenin und Bernsteinsäureanhydrid
   Lit.: G.C. Oliver et al., J.Clin.Invest. 47 (1968), 1035-1042. 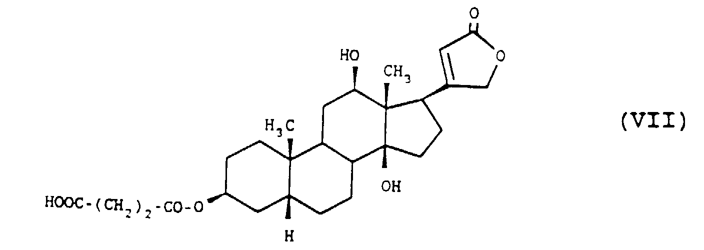
c) Dioxin-4'''-glutaryl-hydroxysuccinimidester
   aus Digoxin und Glutaryl-w-orthotrimethylester-w'-hydroxysuccinimidester in THF.
   Lit.: H.-G. Batz et al., DE-A-2,537,129 bzw. US-4,133,949, US- 4-436,828 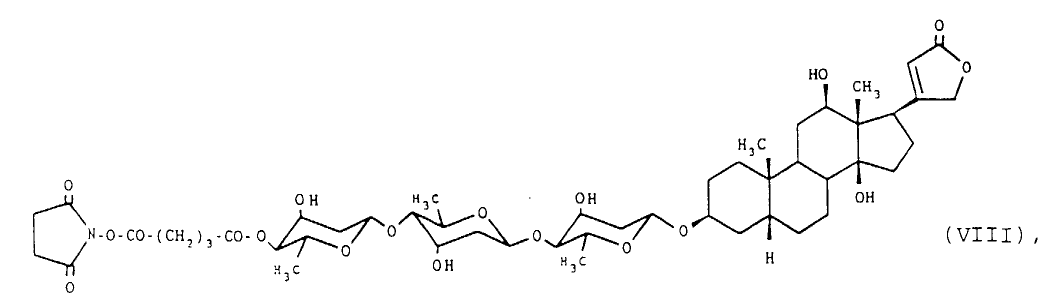
d) 8-(3-Carboxypropyl)-theophyllin
   aus 4,5-Diamino-1,3-dimethylpyrimidin-2,6-dion und Glutansäureanhydrid.
   C.E. Cook et al., Res. Commun. Chem. Pathol. Pharmacol. 13 (1976), 497-505 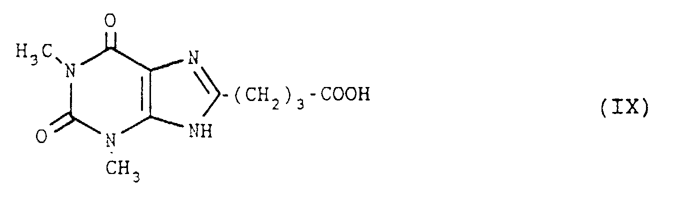
e) 5-Ethyl-5-phenyl-1-(3-carboxypropyl)barbitursäure
   (1-(3-Carboxypropyl)-phenobarbital)
   aus Natriumphenobarbital und 4-Brombuttersäure-ethylester C.E. Cook et al., Quantitative Analogical Studies in Epilepsy P. Kellaway und Ingemar Petersen (ed.), Raven Press, New York 1976, S.39-58.

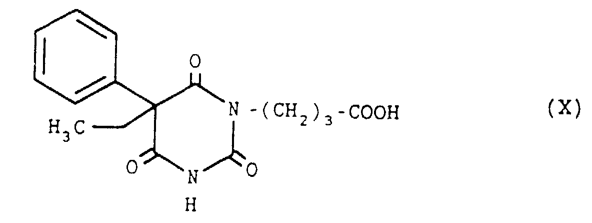

### Beispiel 5 Hapten-Carbonsäure-N-hydroxysaccinimidester

### Allgemeine Herstellvorschrift

0.5 mmol der nach Beispielen 4 a,b,d,e hergestellten HaptenCarbonsäuren werden zusammen mit 63 mg (0.55 mmol) N-Hydroxysuccinimid in 10 ml absolutem DMF gelöst und mit 1.13 mg (0.55 mmol) N,N'-Dicyclohexylcarbodiimid versetzt. Man läßt 4 h bei Raumtemperatur rühren, filtriert und dampft die Lö-sung im Vakuum ein. Der Rückstand wird in 20 ml THF aufgenommen und eventuell unlösliche Reste an N,N'-Dicyclohexylharnstoff durch Filtration entfernt. Die Lösungen der Hapten-Carbonsäure-N-hydroxysuccinimidester werden in dieser Form zur nächsten Stufe eingesetzt.

### Beispiel 6

### Hapten-Carbonsäure-[2-(N-maleinimido)ethylamide]

### Allgemeine Vorschrift:

Zur Lösung der aktivierten Haptene aus Beispiel 5 werden 88 mg (0.5 mmol) 2-(N-maleinimido)ethylamin gegeben. Unter Rühren werden 51 mg (0.5 mmol) Triethylamin zugetropft, danach läßt man 6 h bei Raumtemperatur weiterrühren. Die Lösung wird im Vakuum eingedampft und die Hapten-Maleinimid-Verbindung II A-C durch Säulenchromatographie an einer Kieselgel-Säule (2.5 x 30 cm) mit einem geeigneten Lösungsmittel gereinigt. Die entsprechenden Fraktionen werden gesammelt und im Vakuum eingedampft.

Die Lösung der nach Beispiel 5d hergestellten Verbindungwird im Vakuum auf die Hälfte eingeengt und der Niederschlag abfiltriert. Dann wird das Lösungsmittel im Vakuum vollends entfernt. Der Rückstand wird mit Isopropanol digeriert, das feste Produkt abgesaugt und im Exsikkator getrocknet.

Es werden folgende Produkte erhalten:
a) 2-(N-Maleinimido)ethylamid von Cortisol(3-O-Carboxymethyl)oxim
Eluent: Essigester/Methanol (90/10 v/v).
Ausbeute: 180 mg (64 % d.Th.).
DC:Kieselgel,Essigester/Methanol (90/10 v/v); R_{f} = 0.60.

| | | | | | | | |
|---|---|---|---|---|---|---|---|
| C₂₉H₃₉N₃O₈(557.63); | Ber. | C | 62.46 | H | 7.05 | N | 7.54 |
| | Gef. | C | 62.20 | H | 7.10 | N | 7.31 |

b) 2-(N-Maleinimido)ehtylamid von 3-O-Malonyl-Digoxigenin
Eluent: Essigester/Petrolether/Ethanol (40/40/20 v/v/v).
Ausbeute: 145 mg (47 % d.Th.).
DC:Kieselgel, Essigester/Petrolether/Ethanol (40/40/20 v/v/v); R_{f} = 0.58.

| | | | | | | | |
|---|---|---|---|---|---|---|---|
| C₃₃H₄₄N₂O₉ (612.71); | Ber. | C | 64.69 | H | 7.24 | N | 4.57 |
| | Gef. | C | 64.60 | H | 7.44 | N | 4.29 |

c) 2-(N-Maleinimido)ehtylamid von Digoxin-4'''-glutaryl-hydroxysuccinimidester
Eluent: Essigester/Petrolether/Methanol (40/40/20 v/v/v).
Ausbeute: 220 mg (43 % d.Th.).
DC: Kieselgel, Essigester/Petrolether/Methanol (40/40/20 v/v/v) ;
R_{f} = 0.48.

| | | | | | | | |
|---|---|---|---|---|---|---|---|
| C₅₂H₇₆N₂O₁₈ (1017.15); | Ber. | C | 61.40 | H | 7.53 | N | 2.75 |
| | Gef. | C | 61.31 | H | 7.68 | N | 2.50 |

d) 2-(N-Maleinimido)ethylamid von 8-(3-Carboxypropyl)-theophyllin
Ausbeute: 120 mg (62 % d.Th.).
DC: Kieselgel, Butanol/Eisessig/Wasser (40/10/50 v/v/v); R_{f} = 0.79.

| | | | | | | | |
|---|---|---|---|---|---|---|---|
| C₁₇H₂₀N₆O₅ (388.38); | Ber.: | C | 52.57 | H | 5.19 | N | 21.64 |
| | | C | 52.41 | H | 5.01 | N | 21.74 |

e) 2-(N-Maleinimido)ethylamid von 1-(3-Carboxypropyl)-phenobarbital
Ausbeute: 95 mg (44% d.Th.)
DC: Kieselgel, Essigester/Methanol (90/10 v/v); R_{f}=0.83

| | | | | | | | |
|---|---|---|---|---|---|---|---|
| C₂₂H₂₄N₄O₆ (440.46); | Ber. | C | 59.99 | H | 5.49 | N | 12.72 |
| | | | 59.60 | | 5.79 | | 12.55 |

### Beispiel 7

### Herstellung von Hapten-Peptid-Konjugaten

200 /ug Enzymdonor ED4, beschrieben in US P 4,708,929 (24.11.1987), werden in 150 ml 0.05 m Natriumphosphat-Puffer, pH 6.5, gelöst und mit 20 mg aktiviertem Hapten II A-D IN 20 ml Acetonitril versetzt. Man läßt die Lösung 4 h bei Raumtemperatur rühren. Das Konjugat wird durch HPLC an einer C-18 Säule (Dynamax 60A 8 , 4.6 x 250 mm; Gradient: 20 - 100 % B, B=Acetonitril/Wasser 65/35 v/v) aufgereinigt. Die Retensionszeit des nichtkonjungierten ED4 liegt hierbei bei 25.35 min. Die entsprechenden Fraktionen werden gesammelt und gegen 5 l Wasser dialysiert. Die Lösung des Konjugats kann in dieser Form zu den, in US P 4,708,929 genannten, oder alternativen, immunologischen Testmethoden verwendet werden.

So wurden erhalten:
a) Theopyhllin-8-essigsäure-[2-(N-Maleinimido)ethylamid]-ED4Konjugat
   Ausbeute: 12 /ug; Retensionszeit (HPLC): 26.91 min.
b) Phenobarbital-1-buttersäure-[2-(Maleinimido)ethylamid]-ED4-Konjugat
   Ausbeute: 155 /ug; Retensionszeit (HPLC): 23.78 min.

### Beispiel 8

### Synthese von Cortisol-3-carbonsäure-[2-(N-maleinimido)ethylanid]-β-Gal-Immunogen

500 mg β-Gal (Boehringer Mannheim Nr. 570 079) werden bei 20°C in 50 ml 0.1 n Kaliumphosphatpuffer pH 6.0 gelöst und mit 110 mg der nach Beispiel 4a hergestellten Cortisol-Verbindung in 10 ml Dioxan unter kräftigem Rühren tropfenweise versetzt. Nach beendeter Zugabe läßt man ca. 16 h weiterrühren, dann wird die Lösung auf eine AcA 202 Ultrogel-Säule (800 ml Volumen) aufgegeben und mit 0.01 n Kaliumphosphatpuffer pH 7.0, 0.9% NaCl eluiert. Die proteinhaltigen Fraktionen werden gesammelt und die Konzentration durch UV-Absorption bei 280 nm (Proteinkonzentration = E x 1.9 mg/ml) bestimmt. Die Ausbeute an Immunogen liegt typischerweise bei 350 bis 450 mg. Das Proteinkonjugat wird lyophilisiert und bei -20°C aufbewahrt.

## Patentansprüche

1. Aminoalkylmaleimide der Formel I 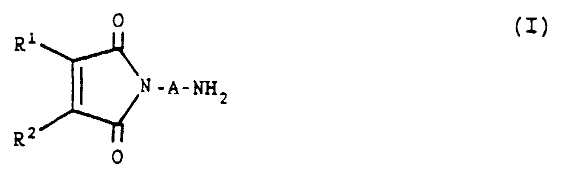 in der R₁ und R₂ gleich oder verschieden sind und Wasserstoff oder eine C₁-C₄-Alkylgruppe bedeuten, und A eine geradkettige oder verzweigte, gesättigte-oder ungesättigte, gegebenenfalls durch ein Sauerstoff- oder Schwefelatom oder eine Carbonylgruppe unterbrochene Alkylengruppe mit 2-6 C-Atomen darstellt, sowie deren Säureadditionssalze, mit Ausnahme der Verbindung N-6-Aminohexylmaleimid.

2. Aminoalkylmaleimide nach Anspruch 1, dadurch gekennzeichnet, daß entweder R₁ oder R₂ Wasserstoff bedeutet.

3. Aminoalkylmaleimide gemäß einem der Ansprüche 1 oder 2, dadurch gekennzeichnet, daß A eine geradkettige Alkylenkette mit 2-5 C-Atomen darstellt.

4. Verfahren zur Herstellung von Aminoalkylmaleimiden nach einem der Ansprüche 1 bis 3, dadurch gekennzeichnet, daß man ein Diamin der allgemeinen Formel III
H₂N-A-NH₂ (III),
in der A die oben angegebene Bedeutung hat, in eine Verbindung der allgemeinen Formel IV
H₂N-A-NH-Y (IV),
überführt, wobei Y eine leicht abspaltbare Schutzgruppe darstellt, und anschließend die Verbindung der allgemeinen Formel IV mit einer Verbindung der allgemeinen Formel V 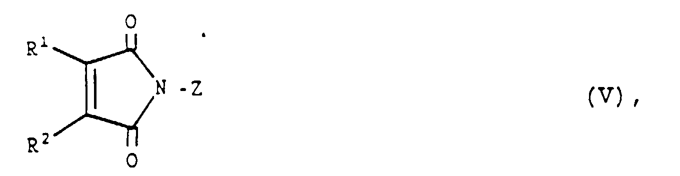 in der R₁ und R₂ die oben angegebenen Bedeutungen haben und Z eine reaktive Gruppe bedeutet, nach an sich bekannten Verfahren umsetzt, und anschließend die Schutzgruppe Y wieder abspaltet.

5. Amidoalkyl-maleimide der allgemeinen Formel II 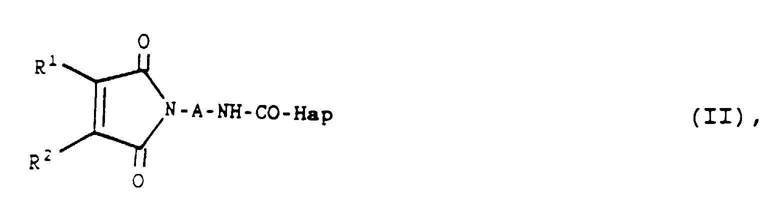 in der Hap der aus einem eine oder mehrere Carboxylgruppen oder Carboxylderivate tragenden Hapten durch Amidierung gebildete Rest ist, wobei das Hapten Phenobarbital, Diphenylhydantoin, Carbamazepin, Valproinsäure, Thyroxin (T4), Triiodthyronin (T3), Östron, Östradiol, Progesteron, Testosteron, Aldosteron, Folsäure, Methyltetrahydrofolsäure oder Cyancobalamin (Vitamin B12), ein Steroidhormon oder ein Xanthinderivat darstellt, R₁ und R₂ gleich oder verschieden sind und eine C₁-C₄-Alkylgruppe oder ein Wasserstoffatom bedeuten, und A eine geradkettige oder verzweigte, gesättigte oder ungesättigte, gegebenenfalls durch ein Sauerstoff- oder Schwefelatom oder Carbonylgruppe unterbrochene Alkylengruppe mit 2-6 C-Atomen darstellt.

6. Verfahren zur Herstellung von Amidoalkylmaleimiden der allgemeinen Formel II nach Anspruch 5, dadurch gekennzeichnet, daß man ein eine oder mehrere Carboxylgruppen oder Carboxylderivate tragendes Hapten, das ausgewählt ist aus Phenobarbital, Diphenylhydantoin, Carbamazepin, Valproinsäure, Thyroxin (T4), Triiodthyronin (T3), Östron, Östradiol, Progesteron, Testosteron, Aldosteron, Folsäure, Methyltetrahydrofolsäure oder Cyancobalamin (Vitamin B12), einem Steroidhormon oder Xanthinderivat, mit einer Verbindung der allgemeinen Formel I gemäß den Ansprüchen 1-3 in an sich bekannter Weise umsetzt.

7. Peptid- oder Proteinkonjugate erhältlich durch Umsetzung von Amidoalkylmaleimiden gemäß Anspruch 5 mit einem eine oder mehrere Sulfhydrylgruppen tragenden Peptid oder Protein.

8. Verfahren zur Herstellung von Konjugaten nach Anspruch 7, dadurch gekennzeichnet, daß man eine Verbindung der allgemeinen Formel II gemäß Anspruch 5 mit einem eine oder mehrere Sulfhydrylgruppen tragenden Peptid oder Protein auf an sich bekannte Weise umsetzt.

9. Verwendung von Verbindungen gemäß einem der Ansprüche 1 - 3 als heterobifunktioneller Linker.

10. Verwendung von Verbindungen der Formel II gemäß Anspruch 5 zur Herstellung von Peptid- oder Proteinkonjugaten.

11. Verwendung von Konjugaten gemäß Anspruch 7 in diagnostischen Bestimmungsverfahren.

12. Diagnostische Mittel enthaltend Konjugate gemäß Anspruch 7 zur Durchführung von immunologischen Bestimmungen.

## Claims

1. Aminoalkylmaleimides of the formula I 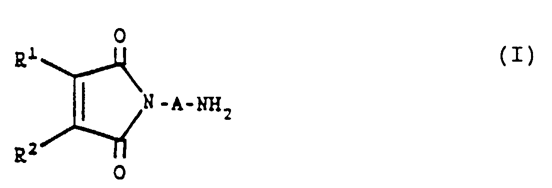 in which R₁ and R₂ are the same or different and denote hydrogen or a C₁-C₄ alkyl group and A represents a straight-chain or branched, saturated or unsaturated alkylene group with 2-6 C atoms which is optionally interrupted by an oxygen or sulphur atom or a carbonyl group, as well as their corresponding acid-addition salts, except the compound N-6-aminohexylmaleimide.

2. Aminoalkylmaleimides as claimed in claim 1, wherein either R₁ or R₂ denotes oxygen.

3. Aminoalkylmaleimides as claimed in one of the claims 1 or 2, wherein A represents a straight-chain alkylene chain with 2-5 C atoms.

4. Process for the production of aminoalkylmaleimides as claimed in one of the claims 1 to 3, wherein a diamine of the general formula III
H₂N-A-NH₂ (III),
in which A has the meaning mentioned above, is converted into a compound of the general formula IV
H₂N-A-NH-Y (IV),
in which Y represents a protecting group which can be easily cleaved off, and subsequently the compound of the general formula IV is reacted according to well-known methods with a compound of the general formula V 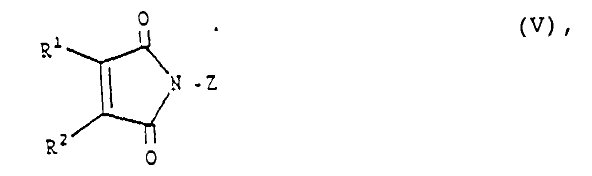 in which R₁ and R₂ have the meanings mentioned above and Z denotes a reactive group and subsequently the protecting group Y is again cleaved off.

5. Amidoalkylmaleimides of the general formula II 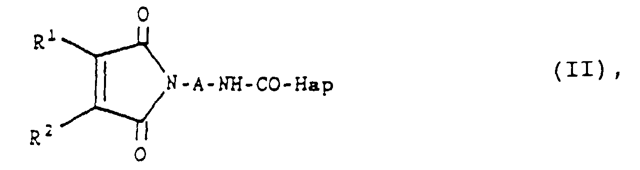 in which Hap is a residue formed by amidation of a hapten carrying one or several carboxyl groups or carboxyl derivatives, the hapten is phenobarbital, diphenylhydantoin, carbamazepine, valproic acid, thyroxine (T4), triiodothyronine (T3), oestrone, oestradiol, progesterone, testosterone, aldosterone, folic acid, methyltetrahydrofolic acid or cyanocobalamin (vitamin B12), a steroid hormone or a xanthine derivative, R₁ and R₂ are the same or different and denote a C₁-C₄ alkyl group or a hydrogen atom and A represents a straight-chain or branched, saturated or unsaturated alkylene group with 2-6 C atoms which is optionally interrupted by an oxygen or sulphur atom or a carbonyl group.

6. Process for the production of amidoalkylmaleimides of the general formula II as claimed in claim 5, wherein a hapten carrying one or several carboxyl groups or carboxyl derivatives, which is selected from phenobarbital, diphenylhydantoin, carbamazepine, valproic acid, thyroxine (T4), triiodothyronine (T3), oestrone, oestradiol, progesterone, testosterone, aldosterone, folic acid, methyltetrahydrofolic acid or cyanocobalamin (vitamin B12), a steroid hormone or a xanthine derivative, is reacted in a well-known manner with a compound of the general formula I according to claims 1-3.

7. Peptide or protein conjugates which are obtained by reaction of amidoalkylmaleimides as claimed in claim 5 with a peptide or protein carrying one or several sulfhydryl groups.

8. Process for the production of conjugates as claimed in claim 7, wherein a compound of the general formula II as claimed in claim 5 is reacted in a well-known manner with a peptide or protein carrying one or several sulfhhydryl groups.

9. Use of compounds as claimed in one of the claims 1-3 as heterobifunctional linkers.

10. Use of compounds of the formula II as claimed in claim 5 for the production of peptide or protein conjugates.

11. Use of conjugates as claimed in claim 7 in diagnostic methods of determination.

12. Diagnostic agent containing conjugates as claimed in claim 7 for carrying out immunological determinations.

## Revendications

1. Aminoalkylmaléimides de formule I 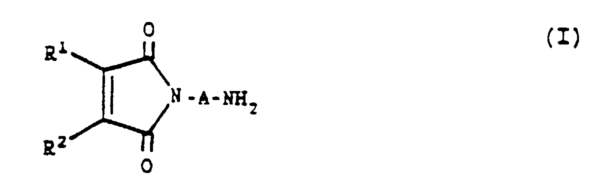 dans laquelle R₁ et R₂ sont identiques ou différents et signifient un atome d'hydrogène ou un groupe alkyle en C₁-C₄ et A représente un groupe alkylène comprenant 2 à 6 atomes de carbone, linéaire ou ramifié, saturé ou insaturé, le cas échéant interrompu par un atome d'oxygène ou un atome de soufre ou un groupe carbonyle ainsi que leurs sels d'addition d'acide, à l'exception du composé N-6-aminohexylmaléimide.

2. Aminoalkylmaléimides selon la revendication 1, caractérisés en ce que R₁ ou R₂ signifie l'atome d'hydrogène.

3. Aminoalkylmaléimides selon l'une quelconque des revendications 1 ou 2, caractérisés en ce que A représente une chaîne alkylène linéaire comprenant 2 à 5 atomes de carbone.

4. Procédé pour la préparation d'aminoalkylmaléimides selon l'une quelconque des revendications 1 à 3, caractérisé en ce qu'on transforme une diamine de formule générale III
H₂N-A-NH₂ (III)
dans laquelle A a la signification indiquée ci-dessus, en un composé de formule générale IV
H₂N-A-NH-Y (IV)
où Y représente un groupe de protection facilement séparable, et en ce qu'on fait ensuite réagir le composé selon la formule générale IV avec un composé de formule générale V 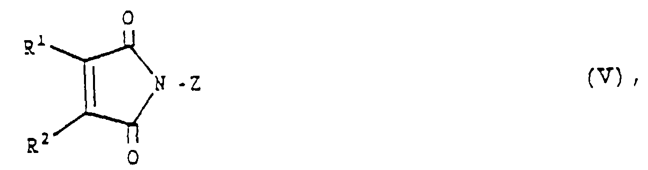 dans laquelle R₁ et R₂ ont les significations indiquées ci-dessus et Z signifie un groupe réactif selon des procédés connus en soi et en ce qu'on sépare ensuite le groupe de protection Y.

5. Aminoalkylmaléimides de formule générale II 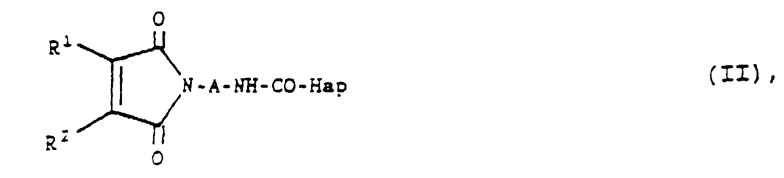 dans laquelle Hap est le résidu formé par amidation à partir d'un haptène portant un ou plusieurs groupes carboxyle ou dérivés carboxyliques, l'haptène représentant du phénobarbital, de la diphénylhydantoïne, de la carbamazépine, de l'acide valproïque, de la thyroxine (T4), de la triiodothyronine (T3), de l'oestrone, de l'estradiol, de la progestérone, de la testostérone, de l'aldostérone, de l'acide folique, de l'acide méthyltétrahydrofolique ou de la cyanocobalamine (vitamine B12), une hormone stéroïdienne ou un dérivé de xanthine, R₁ et R₂ sont identiques ou différents et signifient un groupe alkyle en C₁ à C₄ ou un atome d'hydrogène et A représente un groupe alkylène comprenant 2 à 6 atomes de carbone, linéaire ou ramifié, saturé ou insaturé, le cas échéant interrompu par un atome d'oxygène ou un atome de soufre ou un groupe carbonyle.

6. Procédé pour la préparation d'aminoalkylmaléimides de formule générale II selon la revendication 5, caractérisé en ce qu'on transforme de manière connue en soi un haptène portant un ou plusieurs groupes carboxyle ou dérivés carboxyliques, choisi parmi le phénobarbital, la diphénylhydantoïne, la carbamazépine, l'acide valproïque, la thyroxine (T4), la triiodothyronine (T3), l'oestrone, l'estradiol, la progestérone, la testostérone, l'aldostérone, l'acide folique, l'acide méthyltétrahydrofolique ou la cyanocobalamine (Vitamine B12), une hormone stéroïdienne ou un dérivé de xanthine, avec un composé de formule générale I selon les revendications 1 à 3.

7. Produits conjugués peptidiques ou protéiniques pouvant être obtenus par transformation d'amidoalkylmaléimides selon la revendication 5 avec un peptide ou une protéine portant un ou plusieurs groupes sulfhydryle.

8. Procédé pour la préparation de produits conjugués selon la revendication 7, caractérisé en ce qu'on transforme de manière connue en soi un composé de formule générale II selon la revendication 5 avec un peptide ou une protéine portant un ou plusieurs groupes sulfhydryle.

9. Utilisation de composés selon une quelconque des revendications 1 à 3 comme éléments de liaisons hétérodifonctionnels.

10. Utilisation de composés de formule II selon la revendication 5 pour la préparation de produits conjugués peptidiques ou protéiniques.

11. Utilisation des produits conjugués selon la revendication 7 dans des procédés de détermination diagnostiques.

12. Agents diagnostiques contenant des produits conjugués selon la revendication 7 pour la réalisation de déterminations immunologiques.
